# EUROPEAN PATENT APPLICATION

(11) **EP 3 220 145 A1**
(43) Date of publication of application: **20.09.2017**
(21) Application number: 16160899.7
(22) Date of filing: 17.03.2016
(51) Int. Cl.: G01N 33/68

(54) **IDENTIFICATION OF SUBJECTS AT RISK OF DEVELOPING VENTILATOR-ASSOCIATED PNEUMONIA**

(71) Applicant: Universiteit Antwerpen, 2000 Antwerpen (BE)
(72) Inventor: The designation of the inventor has not yet been filed
(74) Representative: LC Patents

(57) **Abstract**

The present invention relates to the field of ventilator-associated pneumonia, caused by mechanical ventilation of a subject. In particular, it relates to the identification of subjects at risk of developing ventilator-associated pneumonia upon mechanical ventilation of said subject; wherein the identification of said subjects being at risk of developing VAP is based on the determination of one or more interleukins. The present invention also relates to the use of interleukin-reducing compounds for reducing the severity and/or progression of ventilator associated pneumonia in a subject being mechanically ventilated.

SEQUENCE LISTING

<110> Universiteit Antwerpen

<120> IDENTIFICATION OF SUBJECTS AT RISK OF DEVELOPING VENTILATOR-ASSOCIATED
PNEUMONIA

<130> UNA-020

<160> 26

<170> BiSSAP 1.3

<210> 1
<211> 20
<212> DNA
<213> Artificial Sequence

<220>
<223> Primer Sequence

<400> 1
gtcgtaccac tggcattgtg                                                   20


<210> 2
<211> 23
<212> DNA
<213> Artificial Sequence

<220>
<223> Primer Sequence

<400> 2
ctcttttgga ccttgtcgtc ttt                                               23


<210> 3
<211> 21
<212> DNA
<213> Artificial Sequence

<220>
<223> Primer Sequence

<400> 3
ttcctaaagc ccttcaaggc a                                                 21


<210> 4
<211> 20
<212> DNA
<213> Artificial Sequence

<220>
<223> Primer Sequence

<400> 4
cgtcactgac tgtagagagc                                                   20


<210> 5
<211> 23

<212> DNA
<213> Artificial Sequence

<220>
<223> Primer Sequence

<400> 5
atcacacaag accagaagac ttc 23


<210> 6
<211> 22
<212> DNA
<213> Artificial Sequence

<220>
<223> Primer Sequence

<400> 6
attcatgagc atcgccaagt tc 22


<210> 7
<211> 20
<212> DNA
<213> Artificial Sequence

<220>
<223> Primer Sequence

<400> 7
cttctcattc ctgctcgtgg 20


<210> 8
<211> 19
<212> DNA
<213> Artificial Sequence

<220>
<223> Primer Sequence

<400> 8
tgcaggcttc gagatgaac 19


<210> 9
<211> 20
<212> DNA
<213> Artificial Sequence

<220>
<223> Primer Sequence

<400> 9
aagccagagt cattcagagc 20


<210> 10
<211> 20
<212> DNA
<213> Artificial Sequence

<220>
<223> Primer Sequence

<400> 10
cttcaccctg gactctgagc                                                    20


<210> 11
<211> 20
<212> DNA
<213> Artificial Sequence

<220>
<223> Primer Sequence

<400> 11
cccggagacc tctcagaaga                                                    20


<210> 12
<211> 20
<212> DNA
<213> Artificial Sequence

<220>
<223> Primer Sequence

<400> 12
tccagcagcc atacatcgtc                                                    20


<210> 13
<211> 20
<212> DNA
<213> Artificial Sequence

<220>
<223> Primer Sequence

<400> 13
acacacacca gtgggacaaa                                                    20


<210> 14
<211> 20
<212> DNA
<213> Artificial Sequence

<220>
<223> Primer Sequence

<400> 14
ctctcagctg tggtggtgaa                                                    20


<210> 15
<211> 23
<212> DNA
<213> Artificial Sequence

<220>
<223> Primer Sequence

<400> 15
tctccagcat ttgccttaat cgg                                              23

<210> 16
<211> 22
<212> DNA
<213> Artificial Sequence

<220>
<223> Primer Sequence

<400> 16
ggctttctgc actagttgct cg                                               22

<210> 17
<211> 18
<212> DNA
<213> Artificial Sequence

<220>
<223> Primer Sequence

<400> 17
gggctgtcgt tacatccg                                                    18

<210> 18
<211> 22
<212> DNA
<213> Artificial Sequence

<220>
<223> Primer Sequence

<400> 18
aactgggcta cttcgatttt gg                                               22

<210> 19
<211> 24
<212> DNA
<213> Artificial Sequence

<220>
<223> Primer Sequence

<400> 19
tgacagctgg tgaatcactc tgat                                             24

<210> 20
<211> 21
<212> DNA
<213> Artificial Sequence

<220>
<223> Primer Sequence

<400> 20
tgatctgagt gtgagggtct g                                                21

<210> 21
<211> 21
<212> DNA
<213> Artificial Sequence

<220>
<223> Primer Sequence

<400> 21
gggattttgt cgttgcttgt c                                                21


<210> 22
<211> 20
<212> DNA
<213> Artificial Sequence

<220>
<223> Primer Sequence

<400> 22
gtccttagcc actccttctg                                                  20


<210> 23
<211> 20
<212> DNA
<213> Artificial Sequence

<220>
<223> Primer Sequence

<400> 23
tggcggacaa tagaggaaac                                                  20


<210> 24
<211> 20
<212> DNA
<213> Artificial Sequence

<220>
<223> Primer Sequence

<400> 24
gccctacttt ggggttcctc                                                  20


<210> 25
<211> 20
<212> DNA
<213> Artificial Sequence

<220>
<223> Primer Sequence

<400> 25
ggctttgact cctcggaaca                                                  20


<210> 26
<211> 20

<212> DNA
<213> Artificial Sequence

<220>
<223> Primer Sequence

<400> 26
acaaccatca ccacactgga                                                    20

## Description

### FIELD OF THE INVENTION

The present invention relates to the field of ventilator-associated pneumonia, caused by mechanical ventilation of a subject. In particular, it relates to the identification of subjects at risk of developing ventilator-associated pneumonia upon mechanical ventilation of said subject; wherein the identification of said subjects being at risk of developing VAP is based on the determination of one or more interleukins. The present invention also relates to the use of interleukin-reducing compounds for reducing the severity and/or progression of ventilator associated pneumonia in a subject being mechanically ventilated.

### BACKGROUND TO THE INVENTION

Acute pneumonia causes the greatest morbidity and mortality in a hospital setting affecting > 25% of all critically ill patients. Ventilator-associated pneumonia (VAP) developing in patients requiring assisted ventilation constitutes ≈80% of all hospital-acquired pneumonias. Patients receiving mechanical ventilation (MV) for more than 48 hours have a =10-fold higher likelihood of developing VAP compared to non-ventilated patients in intensive care units. Between 250,000 and 300,000 cases per year occur in the United States alone, with incremental costs estimated at between $5,000 and $20,000 per diagnosis. Although several organisms can cause VAP, *Pseudomonas aeruginosa* remains as one of the most important etiologic agent. VAP due to P. *aeruginosa* is also associated with numerous complications such as septic shock and multiple organ dysfunction with mortality rates of up to 40%.

While the precise pathomechanisms involved in development of VAP are unknown, animal models and patient studies have demonstrated that MV causes a marked upregulation of proinflammatory Th1 cytokines such as TNFα, IFNγ, IL6, IL1α and IL1b as well as intense neutrophil migration into the lung in a condition termed ventilator-induced lung injury (VILI). This has been suggested to be the basis of VAP development.
Interestingly, proinflammatory Th1 cytokines have not been shown to corroborate with reduced bacterial clearance and survival. Also peripherally, MV is associated with lowered NK cell activity and lowered IL10 expression in spleen of ventilated rats and a lowered capacity of stimulated blood leukocytes to produce IFNγ, TNFα, and IL6 in children ventilated for 2 h.

Hence, it was an object of the present invention to get a better understanding of the mechanism involved in development of VAP after mechanical ventilation, based on the study of effector cytokines of the main Th cell subsets (Th1, Th2, Th17).

We show here that protective MV of 2-hours in rat leads to an acute upregulation of proinflammatory (Th1) cytokines - IFNγ, IL6, TNFα, and IL1α/b - as observed earlier, but also of several members of IL17 family (IL17A, IL17F, IL22). However, the Th1 cytokines declined rapidly 24-hours post-ventilation and corroborated well with highly downregulated Th1-specific transcription-factor *Tbx21* immediately post-MV, and fits well with published reports of reduced proinflammatory cytokines in peripheral blood cells from children ventilated for 2-hours.

In contrast, we show here a robust MV-induced upregulation of immunosuppressive (Th2) cytokines. While transcript levels of IL4 were elevated to =5-fold immediately after MV (P < 0.001), and remained significantly upregulated 24-hours post-ventilation, IL4 protein levels were also >14-fold elevated in bronchoalveolar lavage fluid 24-hours post-ventilation (P < 0.001). Transcript levels of other Th2 cytokines (IL13 and IL10) were also significantly elevated after MV and associated with increased lung infiltration of immunosupressive, arginase1-positive (Arg1+) M2 macrophages after 24-hours. The Arg1+ macrophages remained significantly elevated when animals were further challenged by intratracheal P. *aeruginosa* inoculation, a known VAP pathogen, where a more severe disease, higher mortality and increased bacterial counts were observed compared to non-ventilated animals that received the same bacterial dose. We propose that Th2 immunosuppressive effects renders MV patients more vulnerable to developing VAP and also co-contribute to the increased morbidity and mortality observed in VAP patients.

In several non-infectious lung inflammatory conditions such as asthma and chronic obstructive pulmonary disease (COPD), upregulation of IL4 and IL13 is observed where it is associated with increased mucus secretion and reduced bacterial immunity. However, despite the potential predetermining effect of MV on VAP development, existence of a similar Th2 cytokine-driven immune-suppression has not been studied before.

Recently, IL17 cytokine family has been shown to play an important role in innate immunity and host defense, however, its role in MV has not been studied. IL17 cytokines could take up both proinflammatory or anti-inflammatory roles depending on the context and have been associated with many inflammatory diseases such as asthma, rheumatoid arthritis, cystic fibrosis, COPD and allograft rejection. Importantly, IL17 also mediate recruitment of neutrophils during bacterial pneumonia. While Th17 cells are major sources of IL17, NKT cells and γδ Tcells are important sources within the innate arm of immunity.

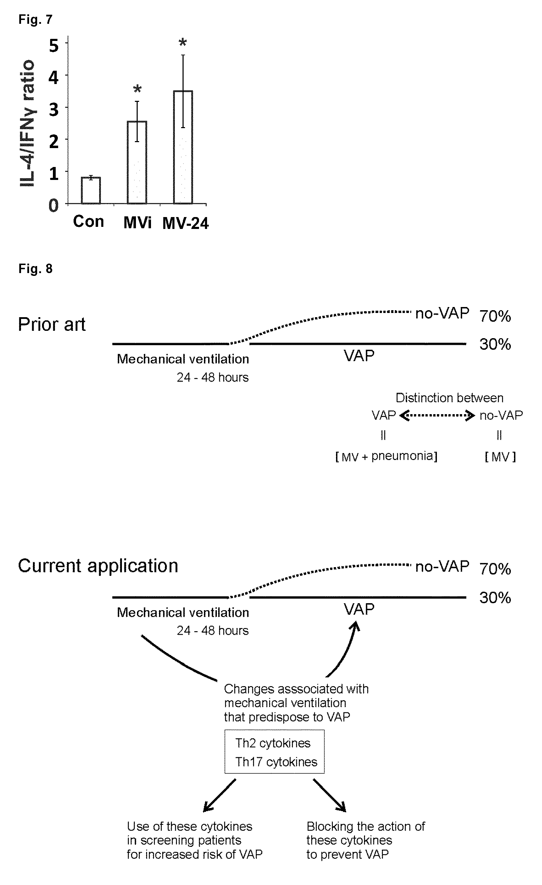

Several publications describe the analysis of interleukin levels for distinguishing VAP from non-VAP after clinical manifestation of VAP-like symptoms such as fever, positive infiltrate on chest radiography and increased inflammatory parameters in blood, in subjects receiving mechanical ventilation (Xia, Liu et al. 2009, Conway Morris, Kefala et al. 2010, Maria Cernada 2015). However, the current invention differs therefrom (see figure 8), in that we have identified several interleukins that are suitable in predicting the risk of developing VAP already shortly after the start of mechanical ventilation, and before the first clinical manifestation of VAP. This is a significant improvement over the currently used methods, in that preventing measures can already be taken before a patient becomes seriously ill due to VAP.

### SUMMARY OF THE INVENTION

In a first aspect the present invention provides a method for detecting a subject at risk of developing ventilator associated pneumonia (VAP), upon mechanical ventilation of said subject; said method comprising the steps of:
a) analysing the expression level of one or more Th2/Th17 cytokines selected from the list comprising IL17a/f, IL4, IL13 and IL22 in a test sample obtained from said subject;
b) comparing the expression level obtained in step a) with the expression level of said one or more Th2/Th17 cytokines in a reference sample;
wherein an increased level in said one or more Th2/Th17 cytokines in said test sample, compared to said control sample, is indicative of a subject having an increased risk of developing ventilator associated pneumonia upon mechanical ventilation of said subject.

In a particular embodiment, said expression level is the absolute expression level of said Th2/Th17 cytokines. In another particular embodiment, said expression level is the relative expression level of said Th2/Th17 cytokines vs one or more Th1 reference cytokines selected from the list comprising IFNγ or IL6.

In a particular embodiment of the present invention, said test sample is obtained within about 1 h to about 48h after the start of mechanical ventilation of said subject; preferably within about 4h to about 24h after the start of mechanical ventilation of said subject. Preferably, said test sample is obtained before the first clinical manifestation of VAP, and after the start of mechanical ventilation of said subject.

In another particular embodiment, said reference sample, is a sample obtained from the subject before the start of mechanical ventilation of said subject.

In a particular embodiment, said samples are selected from the list comprising bronchoalveolar lavage fluid, endotracheal aspirates, whole blood, plasma and serum.

In another particular embodiment, the expression level of said one or more interleukins is determined by protein assays or real-time quantitative PCR assays.

In a further aspect, the present invention provides an interleukin-reducing compound for use in preventing or delaying onset of ventilator associated pneumonia in a subject undergoing mechanical ventilation; wherein said interleukin-reducing compound is selected from the list comprising:
- a compound capable of reducing expression levels of one or more interleukins selected from the list comprising IL17a/f, IL13, IL4,and IL22;
- a compound capable of blocking receptors or co-receptors of one or more interleukins selected from the list comprising IL4, IL13, IL17a/f and IL22; or
- a combination thereof.

The present invention also provides a method for preventing or delaying onset of ventilator associated pneumonia, upon mechanical ventilation of said subject; said method comprising administering to said subject an interleukin-reducing compound selected from the list comprising:
- a compound capable of reducing expression levels of one or more interleukins selected from the list comprising IL4, IL13, IL17a/f and IL22;
- a compound capable of blocking receptors or co-receptors of one or more interleukins selected from the list comprising IL4, IL13, IL17a/f and IL22; or
- a combination thereof.

In a particular embodiment of the uses and methods according to the present invention, said interleukin-reducing compound is selected from the list comprising interleukin-reducing drugs, interleukin-neutralizing antibodies, and interleukin or interleukin receptor antagonists.

In a further embodiment of the uses and methods according to the present invention, said interleukin-reducing compound is administered by intravenous injection and/or via aerosol.

In yet a further embodiment of the uses and methods according to the present invention, said compound is administered within 4h to 48h after the start of mechanical ventilation of said subject, preferably within 4h to 24h after the start of mechanical ventilation of said subject.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

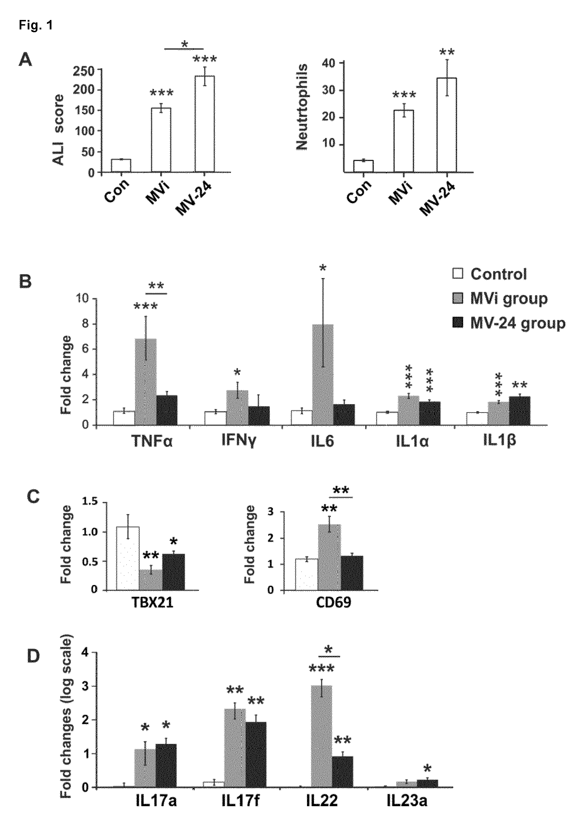

### Figure 1. Mechanical ventilation leads to acute lung injury associated with acute elevation of Th1 cytokines in the absence of Th1 cell stimulation

**A**. Gross lung pathology on H&E stained paraffin sections showed marked signs of pulmonary inflammation with MVi and MV-24 groups characterized by interstitial neutrophils and alveolar septal thickening with MV-24 group showing more proteinaceous debris in airspaces (data not shown). Acute lung injury was evaluated on 6 high magnification images (400x) per slide using pathological scoring scheme modified from the American Thoracic Society. Average amount of neutrophils was counted manually on 200x magnified H&E stained sections and data validated using immunohistochemistry for anti-neutrophil primary antibody. Amount of neutrophils was increased in both MVi (5 times higher) and MV-24 group (8 times higher) compared with control group (***P* < 0.01; ****P* < 0.001). Data is presented as group averages ± SEM, n = 6 animals per group.
**B**. Lung transcript analyses for main Th1 related cytokines show upregulation of *TNF*α (6.7 fold, ****P* < 0.001), *IL6* (8 fold, **P* < 0.05) and *IFNγ* (2.5 fold, *P* < 0.05), *IL1α* (2.3 fold, *P* < 0.001) and *IL1β* (1.9 fold, *P* < 0.001) in MVi group (n = 12) compared to controls (n = 14). At 24h time point (n = 12), TNF lung transcript levels declined 3.1 (***P* < 0.01) compared to MVi group and levels of *IFNγ* and *IL6* returned to pre-MV levels. *IL1α* and *IL1β* remained elevated at 24h time point to the same extend as for MVi group. Data is presented as fold differences relative to healthy control group ± SEM.
**C**. *TBX21* expression in the lungs significantly decreased in both MVi (0.38 fold reduced, ***P* < 0.01) and MV-24 (0.56 fold reduced, **P* < 0.05) groups compared to controls (fold differences ± SEM, n=6 animals per group). Expression of *CD69,* is increased in the MVi group by 2.5 fold compared with healthy controls (*P* < 0.01) and also significantly higher compared with MV-24 group (*P <* 0.01).
**D**. *IL17A* and *IL17F* upregulated by MV with *IL17A* 13.1 fold increased for MVi and 18.3 fold for MV-24 group (**P* < 0.05 for both). *IL17F* was 212 fold increased for MVi and 86 fold for MV-24 group (***P* < 0.01 for both). *IL23a* was 1.5 fold increased at MVi time point although just not significant (*P* = 0.07) but was 1.7 fold significantly increased at MV-24 time point (*P* < 0.05). *IL22* was elevated at MV-24 time point (8.3 fold, *P* < 0.01) and became highly elevated at MVi time point (1051 fold, ****P* < 0.001). Data is presented as fold differences relative to healthy control group ± SEM.

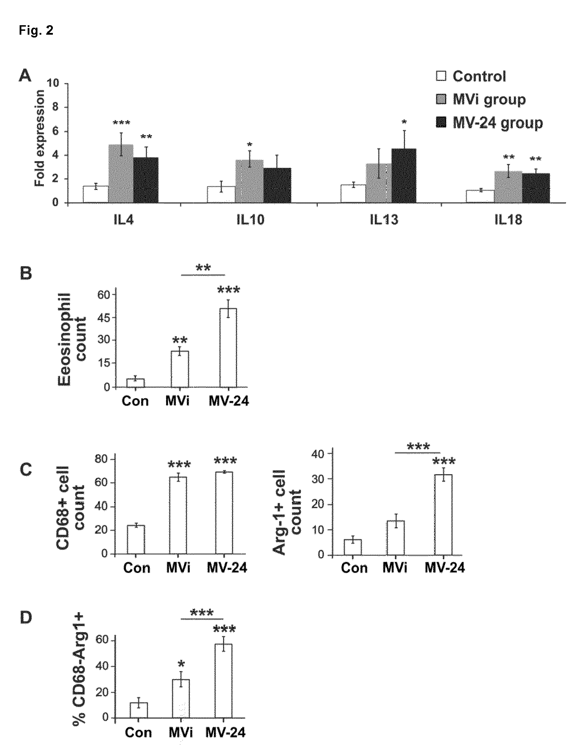

### Figure 2. Mechanical ventilation induces a Th2-related anti-inflammatory response in the lung

**A**. Lung transcript analyses for Th2 related cytokines showed significant upregulation (MVi group, n = 12) of *IL4, IL10* and *IL18* immediately after 2-hours of MV by 4.8 fold (****P* < 0.001), 3.7 fold (**P* < 0.05) and 2.7 fold (***P* < 0.01) respectively. *IL13* was elevated but highly variant in MVi group. Animals from MV-24 group (n = 12) showed sustained levels of *IL4* and *IL18* in the lung (3.7 fold, *P* < 0.05 and 2.7 fold, *P* < 0.01 respectively). Additionally, the trend of elevated *IL13* was more pronounced at MV-24 time point (4.7 fold, *P* < 0.05) with *IL10* showing a slight insignificant decrease. Data is presented as fold differences relative to healthy control group ± SEM.
**B**. Total amount of eosinophils was manually counted on 200 x magnified H&E stained sections (not shown) showing increased eosinophilic infiltration in the lung both at MVi (*P* < 0.01) with MV-24 group (*P* < 0.001) (n = 6 per group).
**C**. Both MVi group and MV-24 group had increased numbers of positive CD68 positive cells (more than 2 times increased, *P* < 0.001 for both). Arginase-1 immunostaining showed that MV-24 group but not MVi-group had significantly higher numbers Arg-1 positive cells in the lung (5 times elevated compared with control group, ≈ 2 times elevated compared with MVi group; *P* < 0.001 for both).
**D.** Double labelled immunohistochemistry (not shown) for CD68 and Arg1 shows significantly higher double positive macrophages in the lung in the MV-24 group and MVi group compared to controls (**P* < 0.05; ****P* < 0.001; n = 6 animals per group).

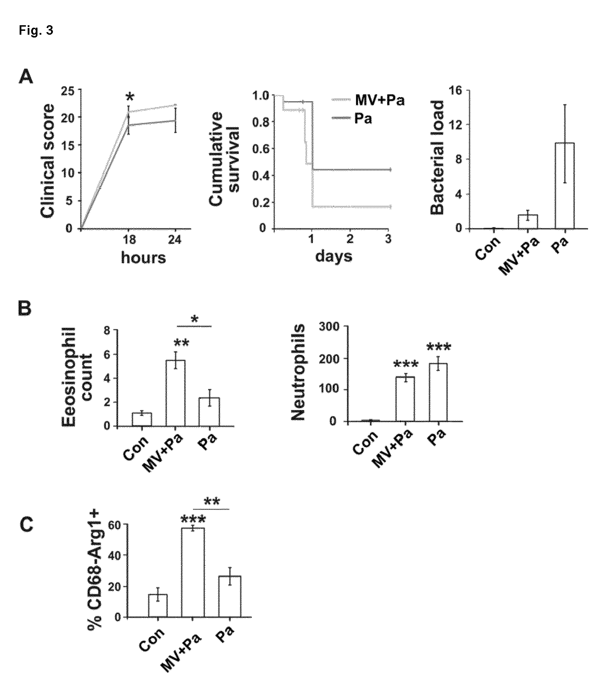

### Figure 3. Bacterial challenge after mechanical ventilation leads to persistence of M2 macrophages and a worse disease outcome

**A.** Clinical scores for both pneumonia models performed using our modified clinical scoring scheme. Both MV+Pa and Pa groups show a progressive pneumonia with the MV+Pa group progressing more rapidly compared to Pa group 18h post infection (**P* < 0.05, 2-tailed *t*-test). Mortality analysis was performed using Kaplan-Meier estimator. Mortality in the MV+Pa group (n=18) was significantly increased compared with Pa group (n=20) (*P* < 0.05, Mantel-Cox log rank test). Data was analyzed using SPSS v21 (IBM). Bacterial load in lungs estimated using qPCR, shows a clear trend towards a higher bacterial load in the MV+Pa group compared to the Pa group.
**B.** General lung pathology showed comparable phenotype for both MV+Pa and Pa groups representing lobar pneumonia with elevated numbers neutrophilic infiltration (****P* < 0.001). Alveolar neutrophils were counted manually by blinded investigator in H&E stained sections (not shown) and results validated by immunohistochemistry using anti-neutrophil antibody. Data is presented as group average with standard error of the means. Manually counted eosinophilic lung infiltrates on H&E stained sections (not shown) showed that MV+Pa group had significantly higher numbers of eosinophils compared with Pa group (2.3 times increased, *P* < 0.05).
**C.** Double-labelled immunohistochemistry revealed that the proportion of CD68+/Arg1+ cells was significantly higher in MV+Pa group (57%) compared with Pa group (26.5%) (*P* < 0.01).

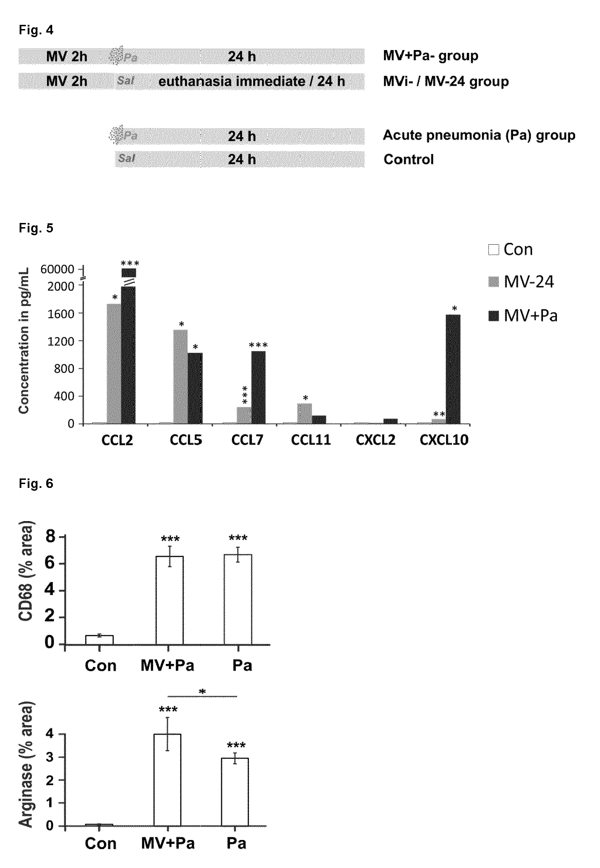

### Figure 4. Experimental setup and models

Graphical presentation of the experimental models employed in the study. VAP-group (MV+Pa) consisted of animals receiving mechanical ventilation followed by intra-tracheal instillation of approx. 2E7 CFU in saline. MVi and MVi injurious groups are animals that receive mechanical ventilation for 2h followed by immediate euthanasia. MV-24-group are animals that received mechanical ventilation for 2 h and euthanasia at 24h time point. Acute pneumonia (Pa) group are animals that receive *P*. *aeruginosa* in saline without prior mechanical ventilation.

### Figure 5. Cytokine and chemokine analyses on BAL in MV-24 group

BAL (broncho-alveolar lavage fluid) cytokine and chemokine analyses on MV-24 group showed significant increased macrophage chemotactic molecules compared with healthy controls.

### Figure 6. Increased Macrophage infiltration and macrophage subtyping for MV+Pa and Pa group

CD68 and Arginase-1 stained sections of MV+Pa and Pa groups were analyzed in a blinded automated way using Image J and presented as the average percentage area stained of each animal within each group with standard error of the mean. Differences between 2 groups are calculated using t-test. Quantitative image analyses showed comparable amounts activated CD68+ macrophages between MV+Pa and Pa groups, both significantly higher compared with healthy controls. Quantitative image analyses of Arg1+ M2 macrophages in MV+Pa group showed higher amounts compared with Pa group (*P* < 0.05).

### Figure 7. Ratio Th2/Th1 cytokines in lungs

For each animal, the ratio IL4 (Th2 cytokine) and IFNγ (Th1 cytokine) was made. Data is presented as group averages ± SEM for 6 animals per group. The ratio of IL4/IFNγ significantly increased after MV (**P* < 0.05 for both MVI and MV-24 groups).

### Figure 8. Comparison with prior art

Scheme representing a comparison of the current invention with the prior art, showing that the prior art mainly focuses on distinguishing VAP from non-VAP in patients which already have clinical manifestations of VAP; whereas the current invention allows to very early determine whether a subject is at risk of developing VAP upon mechanical ventilation, thereby allowing intervention even before the first clinical manifestation of VAP.

### DETAILED DESCRIPTION OF THE INVENTION

As already indicated herein before, the present invention is based on the finding that the levels of certain interleukins are predictive of the risk of developing Ventilator Associated Pneumonia (VAP), already in very early stages after the start of mechanical ventilation.

Hence, in a first aspect the present invention provides a method for detecting a subject at risk of developing ventilator associated pneumonia (VAP), upon mechanical ventilation of said subject; said method comprising the steps of:
a) analysing the expression level of one or more Th2/Th17 cytokines selected from the list comprising IL17a/f, IL4, IL13 and IL22 in a test sample obtained from said subject;
b) comparing the expression level obtained in step a) with the expression level of said one or more Th2/Th17 cytokines in a reference sample;
wherein an increased level in said one or more Th2/Th17 cytokines in said test sample, compared to said control sample, is indicative of a subject having an increased risk of developing ventilator associated pneumonia upon mechanical ventilation of said subject.

As used herein, the term "Ventilator Associated Pneumonia" or VAP is meant to be a type of lung infection that occurs in people who are on breathing machines (i.e. mechanical ventilation) in hospitals. VAP typically affects critically ill persons that are in an intensive care unit and is a major source of increased illness and death. Persons with VAP typically have increased lengths of ICU hospitalization and have up to 20-30% death rate. The current diagnosis usually requires a new infiltrate on chest x-ray plus two or more other factors, such as aberrant body temperature, high white blood cell count, secretions from the airways in the lung, and/or reduction in gas exchange.

The microbiologic flora responsible for VAP is different from that of the more common community-acquired pneumonia (CAP). In particular, viruses and fungi are uncommon causes in people who do not have underlying immune deficiencies. Although any microorganism that causes CAP can cause VAP, there are several bacteria which are particularly important causes of VAP because of their resistance to commonly used antibiotics. These bacteria are referred to as multidrug resistant (MDR):
*Pseudomonas aeruginosa* is the most common MDR Gram-negative bacterium causing VAP. *Pseudomonas* has natural resistance to many antibiotics and has been known to acquire resistance to every antibiotic except for polymyxin B.
*Klebsiella pneumoniae* has natural resistance to some beta-lactam antibiotics such as ampicillin. Resistance to cephalosporins and aztreonam may arise through induction of a plasmid-based extended spectrum beta-lactamase (ESBL) or plasmid-based ampC-type enzyme.
*Serratia marcescens* has an ampC gene which can be induced by exposure to antibiotics such as cephalosporins. Thus, culture sensitivities may initially indicate appropriate treatment which fails due to bacterial response.
*Enterobacter* as a group also have an inducible ampC gene. Enterobacter may also develop resistance by acquiring plasmids.
*Citrobacter* also has an inducible ampC gene.
*Stenotrophomonas maltophilia* often colonizes people who have tracheal tubes but can also cause pneumonia. It is often resistant to a wide array of antibiotics but is usually sensitive to co-trimoxazole.
*Acinetobacter* are becoming more common and may be resistant to carbapenems such as imipenem and meropenem.
*Burkholderia cepacia* is an important organism in people with cystic fibrosis and is often resistant to multiple antibiotics.
*Methicillin-resistant Staphylococcus aureus* is an increasing cause of VAP. As many as fifty percent of *Staphylococcus aureus* isolates in the intensive care setting are resistant to methicillin. Resistance is conferred by the mecA gene.

In the context of the present invention, the term "mechanical ventilation" is to be understood as being a method to mechanically assist or replace spontaneous breathing in a subject in need thereof. This typically involves a machine called a ventilator. Mechanical ventilation mostly involves a tube penetrating through the mouth (such as an endotracheal tube) or the skin (such as a tracheostomy tube). There are two main modes of mechanical ventilation: positive pressure ventilation, where air (or another gas mix) is pushed into the trachea, and negative pressure ventilation, where air is, in essence, sucked into the lungs.

In the context of the present invention the herein mentioned interleukins, i.e. Th2/Th17 cytokines are meant to be understood as follows:
**Interleukin 4 (IL4)** (Genbank accession N° NM_172348.2 and NM_000589.3) is a cytokine that induces differentiation of naive helper T cells (Th0 cells) to Th2 cells. Upon activation by IL-4, Th2 cells subsequently produce additional IL-4 in a positive feedback loop. It is closely related and has functions similar to Interleukin 13. It has many biological roles, including the stimulation of activated B-cell and T-cell proliferation, and the differentiation of B cells into plasma cells. It is a key regulator in humoral and adaptive immunity. IL-4 induces B-cell class switching to IgE, and up-regulates MHC class II production. IL-4 decreases the production of Th1 cells, macrophages, IFN-gamma, and dendritic cell IL-12.
**Interleukin 13 (IL-13)** (Genbank accession N° NM_002188.2) is a cytokine that shares 30% of sequence similarity and structure with is a protein that in humans is encoded by the *IL13* gene. IL-13 was first cloned in 1993 and is located on chromosome 5q31 with a length of 1.4kb. IL-13 and IL-4. It is chiefly secreted by Th2 cells exhibit a 30% of sequence similarity and have a similar structure. IL-13 is a cytokine secreted by many cell types, but especially T helper type 2 (Th2) cells, that is a mediator of allergic inflammation and disease. IL-13 and has effects on immune cells that are similar to those of the closely related cytokine IL-4. However, IL-13 is suspected to be a more central mediator of the physiologic changes induced by allergic inflammation in many tissues.
**Interleukin 17A (IL-17** or **IL-17A)** (Genbank accession N° M_002190.2), originally identified as a transcript from a rodent T-cell hybridoma, is the founding member of a group of cytokines called the IL-17 family. Interleukin 17 is a cytokine that acts as a potent mediator in delayed-type reactions by increasing chemokine production in various tissues to recruit monocytes and neutrophils to the site of inflammation, similar to Interferon gamma. IL-17 is produced by T-helper cells and is induced by IL-23 which results in destructive tissue damage in delayed-type reactions. Interleukin 17 as a family functions as a proinflammatory cytokine that responds to the invasion of the immune system by extracellular pathogens and induces destruction of the pathogen's cellular matrix.
**Interleukin 17F (IL-17F)** (Genbank accession N° NM_052872.3 and XM_011514276.1), is a Th17 cytokine of which 2 isoforms exist. Both have high sequence similarity with IL17A and have convergent functions. Similarly to IL17A, IL17F has potent neutrophil and monocyte attractive functions marking it a potent mediator of inflammation. Combined with IL17A, IL17F has been shown to be an important factor in immune response against extracellular pathogens. IL17F is mostly secreted by Th17 cells, although innate immune cells can secrete IL17F as well during the early stages of inflammation.
**Interleukin 22 (IL-22)** (Genbank accession N° NM_020525.4), is a member of a group of cytokines called the IL-10 family or IL-10 superfamily (including IL-19, IL-20, IL-24, and IL-26), a class of potent mediators of cellular inflammatory responses. IL-22 is produced by activated DC and T cells and initiates innate immune responses against bacterial pathogens especially in epithelial cells such as respiratory and gut epithelial cells. IL-22 along with IL-17 is rapidly produced by splenic LTi-like cells and also produced by Th17 cells and likely plays a role in the coordinated response of both adaptive innate immune systems, autoimmunity and tissue regeneration.

The present invention provides the analysis of the expression level of one or more interleukins, in particular Th2/Th17 cytokines selected from the list comprising: IL17a/f, IL4, IL13 and IL22. It is to be understood that such analysis may include the detection of only one of these interleukins, such as IL17a/f, IL4, IL13 or IL22. However, it may also include the detection of two of these interleukins in combination such as for example IL17a/f and IL4; IL17a/f and IL13; IL17a/f and IL22; IL4 and IL13; IL4 and IL22; IL13 and IL22. On the other hand, it may also include the detection of three of these interleukins in combination such as for example: IL17a/f, IL4 and IL13; IL17a/f, IL4 and IL22; IL17a/f, IL13 and IL22; IL4, IL13 and IL22. Finally, the analysis of the expression level of the interleukins according to the present invention, may also include the analysis of all interleukins in combination, i.e. IL17a/f, IL4, IL13 and IL22.

With the "expression level" of said one or more interleukins of the invention is meant: the levels of interleukins as determined by any suitable method, such as for example by protein assays (e.g. ELISA, Luminex, Mesoscale) or PCR assays such as real-time quantitative PCR assays. In a particular embodiment, said expression level is meant to be the absolute expression level of said interleukins as determined using any suitable method. However, the expression level may also be the relative expression level of said interleukins, i.e. normalized against a housekeeping interleukin, such as for example against a Th1 reference interleukin selected from the list comprising IFNγ or IL6. In said embodiment, the ratio of one or more Th2/Th17 cytokines vs one or more Th1 cytokines may be determined both in the test sample as well as in the reference sample and an increased ratio of these cytokines in the test sample, is then considered to be indicative of a subject at risk of developing VAP upon mechanical ventilation. Evidently in the context of the present invention, only the absolute expression levels, only the relative expression levels (ratio Th2/Th17 vs Th1) or both the absolute and relative expression levels of the Th2/Th17 cytokines of the present invention may be determined.

In particular, said expression level is determined in a test sample obtained from a subject, which is being mechanically ventilated, and the reference (or control) sample is obtained from a subject, which is not being mechanically ventilated. Said reference sample is preferably obtained from the same subject as the test sample; wherein the reference sample is obtained before the start of mechanical ventilation, and the test sample is obtained after the start of mechanical ventilation.

A comparison of the (absolute and/or relative) expression levels of the interleukins of the present invention between the test sample and the reference sample, allows identifying the difference in expression level of said interleukins in both samples. An increase in said interleukins in said test sample compared to said reference sample is indicative of a subject being at risk of developing VAP upon mechanical ventilation of said subject. A relative increase in said interleukin expression levels in particular means a relative increase in the ratio of the said interleukins relative to IFNγ, IL6 and other proinflammatory cytokines expression in said test sample compared to said reference sample.

We have in particular found that already very early after the start of mechanical ventilation the interleukins of the present invention start rising in the event that the subject is at risk of developing VAP later on. Hence, in a particular embodiment, said test sample is obtained within about 1 h to about 48h after the start of mechanical ventilation of said subject; preferably within about 4h to about 24h after the start of mechanical ventilation of said subject. Preferably, said test sample is obtained before the first clinical manifestation of VAP, and after the start of mechanical ventilation of said subject. Evidently, where the reference sample is a sample obtained from the same subject, the reference sample is obtained before the start of mechanical ventilation of said subject.

The samples of the present invention are preferably selected from the list comprising bronchoalveolar lavage fluid, endotracheal aspirates, whole blood, plasma and serum.

While the present invention is in particular very suitable for predicting the risk of developing VAP in a subject being mechanically ventilated, it may of course also be very useful in monitoring the severity and/or evolution of the VAP after it has already set in.

The (very) early prediction of subjects at risk of developing VAP upon mechanical ventilation is highly relevant in the field, since it allows a very early medical intervention, and significantly increases a patients chances of survival and recovery. Once a patient is identified as being at risk of developing VAP, a preventive antibiotic treatment may already be started before the first VAP symptoms even occur. On the other hand, we herewith present a further treatment option for these patients by providing specific interleukin-reducing compounds, to re-establish normal level of the interleukins of the present invention.

Hence, in a further aspect, the present invention provides an interleukin-reducing compound for use in preventing or delaying onset of ventilator associated pneumonia in a subject undergoing mechanical ventilation; wherein said interleukin-reducing compound is selected from the list comprising:
- a compound capable of reducing expression levels of one or more interleukins selected from the list comprising IL4, IL13, IL17a/f and IL22;
- a compound capable of blocking receptors or co-receptors of one or more interleukins selected from the list comprising IL4, IL13, IL17a/f and IL22; or
- a combination thereof.

The present invention, also provides a method for preventing or delaying onset of ventilator associated pneumonia, upon mechanical ventilation of said subject; said method comprising administering to said subject an interleukin-reducing compound selected from the list comprising:
- a compound capable of reducing expression levels of one or more interleukins selected from the list comprising IL4, IL13, IL17a/f and IL22;
- a compound capable of blocking receptors or co-receptors of one or more interleukins selected from the list comprising IL4, IL13, IL17a/f and IL22; or
- a combination thereof.

The present invention further provides a method for preventing or delaying onset of ventilator associated pneumonia, upon mechanical ventilation of said subject; said method comprising the steps of:
a) analysing the expression level of one or more interleukins selected from the list comprising IL17a/f, IL4, IL13 and IL22 in a test sample obtained from said subject;
b) comparing the expression level obtained in step a) with the expression level of said one or more interleukins in a reference sample;
c) identifying a subject having an increased risk of developing ventilator associated pneumonia upon mechanical ventilation of said subject; based on an increased expression level of one or more of said interleukins in comparison to said reference sample;
d) treating said subject of step c) with an interleukin-reducing compound selected from the list comprising:
   - a compound capable of reducing expression levels of one or more interleukins selected from the list comprising IL4, IL13, IL17a/f and IL22;
   - a compound capable of blocking receptors or co-receptors of one or more interleukins selected from the list comprising IL4, IL13, IL17a/f and IL22; or
   - a combination thereof.

Similar, as for the detection assay describe herein above, also for the use in preventing or delaying onset of VAP, any combination of one or more of the interleukins of the present invention may be used.

The term "preventing" is meant to be understood as causing the clinical symptoms of the disease state (i.e. VAP) not to develop in a subject that may be exposed to or predisposed to the disease state, but does not yet experience or display symptoms of the disease state. The term "delaying the onset" is meant to be to as causing the clinical symptoms of the disease state (i.e. VAP) to develop at a later time point in a treated versus a non-treated subject. It may not only include the postponement of the development of symptoms, but it may also include the reduction of the severity of symptoms.

The present invention includes the use of interleukin-reducing compounds for preventing or reducing the onset of VAP in a subject being mechanically ventilated. Said interleukin-reducing compounds may be selected from:
- a compound capable of reducing expression levels of one or more interleukins selected from the list comprising IL4, IL13, IL17a/f and IL22;
- a compound capable of blocking receptors or co-receptors of one or more interleukins selected from the list comprising IL4, IL13, IL17a/f and IL22; or
- a combination thereof.

Said interleukin-reducing compounds are preferably selected from the list comprising interleukin-reducing drugs, interleukin-neutralizing antibodies, and interleukin or interleukin receptor antagonists; and they are used at a (therapeutically) effective amount. As used herein, the term "effective amount" refers to an amount of a compound, or a combination of compounds, of the present invention effective when administered alone or in combination as an anti-proliferative agent. For example, an effective amount refers to an amount of the compound present in a formulation or on a medical device given to a recipient patient or subject sufficient to elicit biological activity, for example, anti-proliferative activity, such as e.g., anti-cancer activity or anti-neoplastic activity. The combination of compounds optionally is a synergistic combination. Synergy, for example, occurs when the effect of the compounds when administered in combination is greater than the additive effect of the compounds when administered alone as a single agent. In general, a synergistic effect is most clearly demonstrated at sub-optimal concentrations of the compounds. Synergy can be in terms of lower cytotoxicity, or increased anti-proliferative effect, or some other beneficial effect of the combination compared with the individual components.

"A therapeutically effective amount" as used herein means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated. A therapeutically effective amount of one or more of the compounds can be formulated with a pharmaceutically acceptable carrier for administration to a human or an animal. Accordingly, the compounds or the formulations can be administered, for example, via oral, parenteral, or topical routes, to provide an effective amount of the compound. In alternative embodiments, the compounds prepared in accordance with the present invention can be used to coat or impregnate a medical device. "Treating", includes any effect, e.g., lessening, reducing, modulating, or eliminating, that results in the improvement of the condition, disease, disorder, etc. "Treating" or "treatment" of a disease state includes: (1) preventing the disease state, i.e. causing the clinical symptoms of the disease state not to develop in a subject that may be exposed to or predisposed to the disease state, but does not yet experience or display symptoms of the disease state; (2) inhibiting the disease state, i.e., arresting the development of the disease state or its clinical symptoms; or (3) relieving the disease state, i.e., causing temporary or permanent regression of the disease state or its clinical symptoms.

The interleukin-reducing compound is preferably administered by intravenous injection and/or via aerosol. The interleukin-reducing compound may be administered already very early after the start of mechanical ventilation. However, in order to allow sufficient time for the clinicians to determine the interleukin expression status, using the methods of the present invention, the treatment is preferably started within 4h to 48h after the start of mechanical ventilation of said subject, preferably within 4h to 24h after the start of mechanical ventilation of said subject. Preferably, the treatment is started before the first symptoms of VAP start to develop in the subject.

### EXAMPLES

### MATERIAL AND METHODS

### Animal experimentation groups

A total of 131 adult male Wistar rats (mean weight 345 g, SD = 23.5 g, Charles Rivers, Saint-Germain-Nuelles, France) were used in this study of which 46 animals were required for bacterial dose tittering and MV setup optimization. A total of 61 animals were used for survival analyses. A total of 53 animals were further subjected for characterizations and were randomly assigned into one of the 6 groups: **(i)** MVi group where animals received MV for 2 h and were immediately euthanized, (n = 12); **(ii)** MV-24 group where animals received MV for 2 h, instilled with saline and euthanasia at 24 h (n = 12); **(iii)** MV+Pa group where animals after receiving MV for 2 h were immediately instilled with 2 x10⁷ of P. *aeruginosa* and euthanized at 24 h (n = 6); **(iv)** Acute pneumonia model Pa group where animals received same bacterial instillation without prior ventilation and euthanasia after 24h (n = 6); **(v)** Healthy control group (n = 14). Additionally, **(vi)** animals were also ventilated using injurious MV settings (n = 3).

### Endotracheal intubation

Rats were anesthetized using 100 mg/kg ketamine and 1 mg/kg medetomidine and intubated utilizing a Hallowell tilting rat intubation platform (Hallowell EMC, Pittsfield, USA). Briefly, anesthetized rats were placed on the tilting platform in a horizontal position. An elastic band was placed behind the front incisors and attached to the platform. The platform was tilted 45° and the tongue was pulled out using blunt forceps. Next, the vocal cords were visualized using a specially designed wedge mounted on an otoscope and a 14 G catheter was placed into the trachea with the aid of a guide wire. With 14 G catheter animals were mechanically ventilated and/or instilled with saline or measured amounts of free bacteria, extubated, anesthesia antagonized with 300 µg/kg atipamezole, and animals returned to their cages and followed up.

### Mechanical ventilation

For mechanical ventilation, 4 rats were simultaneously ventilated by Servo 900 C ventilator (Siemens, Solna, Sweden) either using "protective ventilation" or "non-protective, injurious ventilation" settings. For a protective MV setting, a pressure controlled mechanical ventilation mode with the following settings was applied: 10 cm H₂O maximum pressure; 4 cm H₂O positive end-expiratory pressure (PEEP); 80 breaths/min respiratory rate; time inspiratory/expiratory (TI/E) 1:2; and fraction of inspired oxygen of 0.21 as utilized previously resulting in ≈8 mL/kg Vt. For aggressive mode, rats were simultaneously ventilated with zero PEEP; 26 cm H₂O of peak inspiratory pressure (PIP); and 40 breaths per minute resulting in ≈30 mL/kg Vt. For both groups, animals were continuously monitored using a pulse oximeter (MouseSTAT, Kent Scientific, Torrington, USA) to control the oxygen saturation and body temperature was maintained at 37°C by use of a rectal temperature probe connected with a heating blanket (RightTEMP Kent Scientific).

### Pneumonia models

For creation of pneumonia models, *P. aeruginosa* ATCC 27853 strain was used that has been extensively utilized for *in vivo* and *in vitro* studies. Animals were inoculated with same dose of *P. aeruginosa* (2E7 CFU) to allow comparisons between both pneumonia models. For creation of VAP model (MV+Pa), animals receiving a protective MV for 2 h were inoculated with 2E7 CFU *P. aeruginosa* in 1 ml saline. Acute pneumonia (Pa) model did not receive MV and were directly instilled with the same dose of *P*. *aeruginosa.* For creation of infectious models, select colonies from overnight culture of *P*. *aeruginosa* ATCC 27853 were diluted in sterile PBS until 0.5 McFarland. Bacterial suspension is diluted approximately 5 times and 500 µL of bacterial suspension is used per animal. Sterile saline was used for control animals. After inoculation, all animals were monitored 3 times per day for clinical signs of pneumonia using a modified scoring scheme **(Table. 1).**

**Table 1: Clinical scoring scheme**

| **Stage disease development** | **Description of clinical symptoms** | **Weight** |
|---|---|---|
| | Avoidance | 1 |
| **Stage 1: early signs of pneumonia** | Loss of appetite | 1 |
| | Fever | 1 |
| | Piloerection | 1 |
| | Rapid breathing | 1 |
| | Ruffled fur | 1 |
| | Labored breathing | 2 |
| **Stage 2: signs of severe inflammation and infection** | Hunched posture | 2 |
| | Wheezing, small cough | 2 |
| | Lethargy | 2 |
| | Periorbital congestion | 2 |
| **Stage 3: signs of compromised lung function** | Very shallow breathing | 3 |
| | Prostrate, not reactive | 3 |
| | Gasping | 3 |
| | Cyanosis at the tip of tail and feet | 3 |

All animals were euthanized with isoflurane overdose at pre-determined time points. After euthanasia, blood was collected by cardiac puncture utilizing EDTA coated tubes. The trachea was exposed and lungs lavaged by a 14-gauge angiocatheter with 20 mL/kg body-weight of ice-cold sterile PBS for rats. Left lung was removed, washed in sterile PBS and snap frozen in liquid nitrogen. Right lung was washed and fixed overnight in 2 % paraformaldehyde and prepared for paraffin embedding.

### Histopathology

Lung pathology scoring was performed on H&E stained 5 µm thick paraffin sections as done previously. Lung pathology was assessed blinded by trained pathologist using modified scoring scheme for acute lung injury adopted from The American Thoracic Society. Amount of neutrophils and eosinophils for each group were manually counted on 10-20 consecutive images grabbed using 200x magnification per slide. For slides that showed heterogenic staining pattern, most affected areas were imaged for quantification.

### Immunohistochemistry

After deparaffinization and rehydration, antigen retrieval was performed in citrate buffer (0.018 M citric acid.H₂O and 0.082 M sodium citrate.H₂O using microwave heating. Endogen peroxidase blocking was performed for immunohistochemistry stained sections by incubating slides for 15 min in 0.3% H₂O₂ followed. Blocking of non-specific antigens was performed by a serum block using 1:5 diluted normal horse serum in 1 % BSA solution for 30 min. All primary antibodies were diluted in PBS-1% BSA solution and incubated overnight at 4°C.
The primary antibodies utilized in the study were: mouse monoclonal anti-CD68 1:200 dilution (Abd Serotec MCA341R, Kidlington, UK), goat polyclonal anti-arginase-1 1:300 dilution (Santa Cruz Sc-18354, Heidelberg, Germany), rabbit monoclonal anti-CD3 1:200 dilution (Abcam ab16669, Cambridge, UK). After washing in PBS, biotin conjugated secondary antibodies (DAG, DAM, both 1:200 dilution, Jackson Immunoresearch, Suffolk, UK) were incubated for 30 min at room temperature, washed and incubated with extravidin conjugated HRP for 30 min. Color development was performed using DAB (5', 5' diaminobenzedine, Dako, Heverlee, Belgium) solution and the reaction stopped by PBS. Images were grabbed with a 5x objective and Olympus UC30 color camera (Olympus, Antwerp, Belgium). ImageJ 1.48v (Scion Corporation, USA) was used for quantification of positively stained area on DAB-stained lung histology slides of the infected animals. Briefly, images were first converted into 16 bit grey scale. Next, positive pixels were segmented by applying automated thresholding using the Yen algorithm. The percentage of positive area was calculated by dividing the total pixel area of positively stained areas by total pixel area corresponding to the total lung tissue in the field of view. The mean percentage of positive stained area then calculated for each animal. All analyses were performed by a blinded investigator. Non infected inflammatory groups were analyzed by manual quantification.

For double immunocytochemistry staining, mouse monoclonal anti-CD68 1:200 dilution (Abd Serotec MCA341R), goat polyclonal anti-arginase-1 1:400 dilution (Santa Cruz Sc-18354) primary antibodies were used. After washing in PBS, sections were incubated for 30 min at room temperature with DAG-cy3 (1:200) and DAM-cy5 (1:200)(Jackson Immunoresearch). After washing, sections were incubated for 5 min in 5 µg/mL DAPI (Sigma-Aldrich, Diegem, Belgium), washed with PBS and sections coverslipped using anti-fading PBS-glycerol (Citifluor, London, UK). High-resolution images were grabbed using a dual spinning disk confocal microscope (Ultra *View* VoX, PerkinElmer, Seer green, UK) and images analyzed using Volocity (Perkin Elmer). Proportion of CD68-Arginase positive cells were manually counted.

### Q-RT-PCR

Total RNA from lung tissue was extracted using RNeasy-mini spin columns (Qiagen, Hilden, Germany) after grinding in liquid nitrogen. RNA integrity and concentrations were estimated using RNA-nanochips on Bio-analyzer (Agilent, Diegem, Belgium). Extracted RNA was converted to cDNA by reverse transcriptase (RT² First Strand Kit, Qiagen). Quantitative PCR was performed using Bio-Rad CFX connect (Bio-Rad, Temse, Belgium) and SsoAdvanced SYBR green supermix (Bio-Rad) using 2 step PCR with cycles of 95°C for 10 second followed by 60°C for 30 seconds. Primer pairs for *ACTB, SDHA*, *IL4, IL13, IFN*γ, *TNF, IL1b, IL22, IL17f, IL23a* were used (for primer sequences, see **Table. 2**)**.**

**Table 2: Primer sequences**

| **Target** | **Host** | **Fw seq** | **SEQ N°** | **Rv seq** | **SEQ N°** |
|---|---|---|---|---|---|
| *A ctb* | Rat | GTCGTACCACTGGCATTGTG | 1 | CTCTCAGCTGTGGTGGTGAA | 14 |
| *Sdha* | Rat | CTCTTTTGGACCTTGTCGTCTTT | 2 | TCTCCAGCATTTGCCTTAATCGG | 15 |
| *Ywhag* | Rat | TTCCTAAAGCCCTTCAAGGCA | 3 | GGCTTTCTGCACTAGTTGCTCG | 16 |
| *IL4* | Rat | CGTCACTGACTGTAGAGAGC | 4 | GGGCTGTCGTTACATCCG | 17 |
| *IL13* | Rat | ATCACACAAGACCAGAAGACTTC | 5 | AACTGGGCTACTTCGATTTTGG | 18 |
| *IFNy* | Rat | ATTCATGAGCATCGCCAAGTTC | 6 | TGACAGCTGGTGAATCACTCTGAT | 19 |
| *TNF* | Rat | CTTCTCATTCCTGCTCGTGG | 7 | TGATCTGAGTGTGAGGGTCTG | 20 |
| *IL1b* | Rat | TGCAGGCTTCGAGATGAAC | 8 | GGGATTTTGTCGTTGCTTGTC | 21 |
| *IL6* | Rat | AAGCCAGAGTCATTCAGAGC | 9 | GTCCTTAGCCACTCCTTCTG | 22 |
| *IL17a* | Rat | CTTCACCCTGGACTCTGAGC | 10 | TGGCGGACAATAGAGGAAAC | 23 |
| *IL17f* | Rat | CCCGGAGACCTCTCAGAAGA | 11 | GCCCTACTTTGGGGTTCCTC | 24 |
| *IL22* | Rat | TCCAGCAGCCATACATCGTC | 12 | GGCTTTGACTCCTCGGAACA | 25 |
| *IL23* | Rat | ACACACACCAGTGGGACAAA | 13 | ACAACCATCACCACACTGGA | 26 |

Data was analyzed using comparative C_{T} method with *ACTB* and *SDHA* as housekeeping genes as described earlier (Schmittgen and Livak 2008). Additional gene expression changes in select animals were analyzed using the commercial Rat Innate and Adaptive Immune Responses RT² Profiler PCR array (Qiagen) and custom PCR-arrays (Qiagen) including following genes of interest: *Lcn2, II17f, II4, CD69, Camp, Crp, II18, II5, Rorc, Ctsb, Ifng, II1a, II6, tbx21, Ctsg, II10, II1b, TgfB1, Hist1h4b, II12a, II12a, 112, Tnf, H2afx, 1113, 1121, Foxp3, Ctsd, II17a, II22, Faslg, Defb4, 1117b, II23a,* and *Gata3*. Housekeeping genes included were: *Ywhag, Actb, Polr2b, Gapdh, Sdha* and *Tbp.* Additionally, a random genomic DNA contamination control, 3 reverse transcriptase control and 3 PCR controls were included. Prior to PCR-array analyses, cDNA quality was validated on qPCR for *Actb, Sdha, Ywhag* and *IL6.* All data was analyzed using comparative C_{T} method as described above.

### Luminex chemokine analyses

Magnetic bead based multiplex immunoassay on BAL samples from MV24 and VAP group for IL4 and select chemokines (CCL11, CXCL10, CCL2, CCL7, CXCL2, CCL5) was performed on Magpix Luminex platform using a custom ProcartaPlex rat panel at manufactures' site (eBiosciences, Vienna, Austria.).

### Bacterial quantification

Because in our pilot experiments, lung pathology observed by intra-tracheal inoculation was equal in both lungs, we opted to take the entire lung for histology and the other lung snap frozen, completely crunched and than subsampled for molecular analyses to avoid a sample bias. As such, microbial estimations were performed using PCR on DNA extracted from whole lung homogenate. For DNA extractions, crushed lung homogenates were lysed according to SDS-proteinase K protocol described earlier. DNA cleanup was performed on crude PCR lysates using spin columns (Nucleospin, Macherey Nagel). Quantitative PCR was performed using Bio-Rad CFX connect (Bio-Rad) and SsoAdvanced SYBR green supermix (Bio-Rad) using 3 step PCR with cycles of 95°C for 15 second followed by 60°C for 30 seconds and 72°C for 30 seconds. Primer pairs are available on request. As control samples, DNA from healthy controls animals was spiked with DNA of pure *P*. *aeruginosa* culture.

### Data analyses and statistics

Data analyses were performed using Microsoft Excel and SPSS version 21 (IBM, USA). Survival analyses were performed using Kaplan Meier estimator with Mantel-Cox log rank test for testing significant differences between the groups. Lung transcript analyses were performed as stated earlier (Schmittgen and Livak 2008). Briefly, the combined average of each of the appropriate control groups was used to calculate the fold differences in the study groups. For testing statistical significance, the fold differences for each gene of animal were log transformed. Data was analyzed using *t*-test for independent samples or using Mann-Whitney U test depending on the distribution of the samples. Where multiple comparisons were necessary, a one-way ANOVA with post-hoc Bonferroni correction was applied. Data is presented as average fold differences with standard error of the mean. Immunohistological and immunocytochemistry data is presented as the mean percentage area of each group with standard error of the mean and differences between groups tested using 2-tailed independent *t*-test.

### RESULTS

### Mechanical ventilation leads to acute lung injury associated with acute elevation of Th1 cytokines in the absence of Th1 cell stimulation

To study whether mechanical ventilation causes local immunosuppressive changes, we mechanically ventilated rats using a protective ventilation protocol to mimic the MV protocols currently being used in humans (see *Methods*)*.* Animals were immediately studied at the end of MV ("MVi" group) or after 24 h of recovery (MV-24 group). Two hours of protective ventilation in rats led to a mild degree of ventilator-induced lung injury (VILI) in both the MVi and MV-24 groups, with histological features of loss of alveolar membrane integrity and presence of inflammatory cellular infiltration (Fig. 1A), as described previously. However, compared to the MVi group, the MV-24 group showed slightly higher lung pathology, specifically higher amounts of fibrin and blood clots Quantitative and manual immunohistochemical studies for CD68-positive cells, a marker of activated macrophages, showed a significant 2-fold upregulation in MVi and MV-24 groups **(****Fig. 2C****)** suggesting that initial phases of VILI indeed involved neutrophilic and macrophage-mediated injury. MV also led to an immediate increase in neutrophilic and macrophage-related proinflammatory cytokines such as *TNF*α (6.7 fold, *P* < 0.001), *IL6* (8 fold, *P* < 0.05), *IFNγ* (2.5 fold, *P* < 0.05), *IL1α* (2.3 fold, *P* < 0.001) and *IL1β* (1.9 fold, *P* < 0.001) **(****Fig. 1B****).** These data are in agreement with previous studies showing that MV induces a Th1 proinflammatory cytokine response.

We questioned whether the proinflammatory response observed immediately after MV has any long-term consequences in building a Th1 proinflammatory milieu. Lung transcript analysis after 24 hours showed that the highly upregulated proinflammatory cytokines observed immediately after MV plateaued or significantly recovered in the MV-24 group with a significant drop in *TNFα* (3.1 fold reduced, *P* < 0.01) and *IFNγ* (2.1 fold reduced, *P* < 0.05), IL6 (5.3 fold reduced, *P* < 0.01), while *IL1α* and *IL1β* lung transcripts were unaltered **(****Fig. 1B****).** These data were also confirmed with another set of gene-specific primers from independently extracted tissue RNA.

Next, we studied the possible cellular sources of Th1 cytokines secreted acutely after MV. The major sources of IFNγ in tissue are NKT and Th1 cells. Once stimulated, Th1 cells also secrete IL2. Interestingly, IL2, unaltered as an immediate consequence of MV, significantly reduced after 24 hours of MV (3.0 fold, *P* < 0.05). The levels of IL12, a potent activator of NKT cells in the secretion of IFNγ, were also unaltered. To study the activation state of resident Th1 cells in the lung, we analyzed the expression of *Tbx21,* a highly specific Th1-specific transcription factor. Surprisingly, the expression of *Tbx21* was reduced by a factor of 2.7 fold (*P* < 0.01) in MVi group of animals compared to healthy control group and was still significantly depressed after 24 hours of recovery (1.8 fold reduced; *P* < 0.05) **(****Fig. 1 C)****.** On the other hand, CD69, a marker for activated T-cells was upregulated by 2.4 fold (P < 0.01) in MVi animals **(****Fig. 1C****)** indicating the involvement of T-cell lineages other than Th1 in the long-term consequences of MV induced lung injury.

### Important role of Th17 cytokines in MV-induced acute lung injury

We next turned our attention to the IL17 family of cytokines whose role has not been explored in MV induced inflammation. IL17, like IFNγ, is a potent mediator in delayed-type proinflammatory reactions by increasing chemokine production to recruit monocytes and neutrophils to the site of inflammation. In fact, in the absence of sustained expression of IFNγ and other Th1 cytokines, recruitment of neutrophils has been shown to be primarily caused by IL17 in allergic lung inflammation (Park, Li et al. 2005). Our identification of a declining Th1 cytokine activity in presence of increasing neutrophils in MV-24 group of animals suggested a similar role of IL17 in MV-associated injury. We showed that both IL17A and IL17F, the two most important Th17 cytokines were upregulated 13-fold or more (*P* < 0.05 and < 0.01 for IL17A and F, respectively) **(****Fig. 1D****).** A similar trend was observed in the MV-24 group with high expression of both IL17A and IL17F (both upregulated more than at least 18 fold, *P* < 0.05 for IL-17a and P < 0.01 for IL17f) **(****Fig. 1 D)****.**

We next similarly questioned whether increased IL17 in MVi group has a long-term consequence in building a Th17 cell-mediated immune milieu and studied IL22 and IL23a. IL22 is an important effector cytokine in the IL17 pathway involved in chronic inflammatory diseases and protection against bacterial infections and was upregulated in both MVi (7 fold) and MV-24 (14.2 fold) groups (*P* < 0.01 for both) **(****Fig. 1D****).** On the other hand, IL23a is a potent activator of Th17 cells in promoting proliferation and survival of Th17 cells and enhancing IL17 production and was 1.6 fold upregulated in MV-24 animals (*P* < 0.05) **(****Fig. 1 D)** and corroborated with 2.1-fold increased expression of Th17-specific transcriptional factor *Rorc* (*P* < 0.01). These data suggest that MV drives IL17 secretion and also leads to Th17 cell differentiation.

### Mechanical ventilation induces a marked Th2 related anti-inflammatory response in the lung with increased eosinophils, arg1+ macrophages and highly upregulated Th2 cytokines

We further studied the role of Th2 cytokines in MV. We first studied IL4, a potent inducer of naive CD4⁺ T cells differentiation into CD4⁺Th2 effector cells and showed it to be significantly upregulated by 4.8 fold in the MVi group (*P* < 0.001), that remained significantly upregulated also in MV-24 animals (3.7 fold, *P* < 0.05) **(****Fig. 2A****).** To study if elevated IL4 transcript levels were also stable translated in tissue, we studied IL4 protein levels in the bronchoalveolar lavage fluid in the MV-24 group of animals and while IL4 levels in all healthy controls were below the detection limit of 0.1 pg/ml, animals in MV-24 group had a highly statistically significant elevation (Average 1.5 ng/ml; SD, 0.92 ng/ml; P < 0.001).

We also investigated the role of IL10 and IL13. IL10 is primarily an immune modulatory cytokine and down regulates the expression of Th1 cytokines and MHC class II molecules. We show that IL10 was also significantly upregulated by 3.7 fold (*P* < 0.05) immediately after MV in the MVi group of animals **(****Fig. 2A****).** On the other hand, IL13 was only significantly elevated after 24 hours in the MV-24 group of animals (4.7-fold upregulated, *P* < 0.05), but not immediately after MV **(****Fig. 2A****).** While having anti-inflammatory properties, IL13 is primarily associated with the induction of airway diseases (Venkayya, Lam et al. 2002). We thus questioned whether a non-protective ventilation setting could cause a heightened IL13 response evident immediately after MV. For this, we ventilated rats with a non-protective ventilation setting (≈30 mL/kg Vt, 0 PEEP and 26 cm H₂O of PIP) and studied IL13 along with prototype proinflammatory cytokines IFNγ and TNFα in the lungs immediately after MV. As expected, animals receiving non-protective ventilation showed a worsened lung pathology marked by increased fibrin and red blood cells in the alveoli and high levels of activated alveolar macrophages. On lung transcript analyses, TNFα was also highly elevated (27 fold, *P* < 0.001) although IFNγ levels were not significantly increased compared to the MVi group receiving protective ventilation. Interestingly, all animals that received the non-protective ventilation showed very high expression of IL13 (≈80 fold, *P* < 0.001) at MVi time point suggesting that IL13 is also an important Th2 cytokine in early VILI pathogenesis.

We further studied the cellular sources of these Th2 cytokines. In the innate arm, NKT cells are the major source of IL4 and IL13 while in the adaptive arm, Th2 cells become the major source. Moreover, eosinophils have long been associated with the effector arm of Th2 immune responses and recent data even indicates that activated eosinophils are present most early in response to Th2-inducing agents where they function in the initiation of Th2 immunity. Interestingly, the numbers of eosinophils were 4-fold elevated in the MVi-group that increased to 7-fold elevation in the MV-24 group compared to healthy controls (*P*-value < 0.001 for both) **(****Fig. 2B****).** Using a magnetic bead assay we also studied key CC and CXC chemokines in relation to MV and showed that CCL11/ Eotaxin, a key eosinophil chemotactic protein, was elevated 42 fold in MV-24 group (*P* < 0.05) along with other eosinophil or macrophage chemokines such as CCL2, CCL5, CCL7 and CXCL10 **(SI** **Fig. 5****).**

We also studied IL18, a macrophage product that induces the development of naive Th0 cells into Th2 cells and also stimulates innate immune cells in the lung in the production of IL4, IL13, and IL1 7(Nakanishi, Yoshimoto et al. 2001). This effect of IL18 is inhibited in the presence of IL12 (Xu, Trajkovic et al. 2000). We showed that IL18 was 2.7-fold upregulated in MVi animals (*P* < 0.01) and also remained elevated at the MV-24 time point (*P* < 0.01) **(****Fig. 2A****)** and fits well with lack of IL12 alteration in MVi or MV-24 group of animals.

To study whether this increased Th2 response has an effector function on macrophages, we studied the presence of arginase 1-positive (Arg1+) immunosuppressive M2 macrophages in MVi and MV-24 animals. While not significantly different in MVi group, a significant increase in Arg1+ M2 macrophages was observed in the MV-24 group compared to healthy control (*P* < 0.001) **(****Fig. 2C****).** The increased infiltration of Arg1+ macrophages as a consequence of MV was also confirmed by quantitative CD68/arginase1 double immunocytochemistry showing that despite strong acute concurrent Th1 cytokine activation, 57% of activated macrophages in MV-24 animals were Arg1+ M2 macrophages **(****Fig. 2D****)** compared to 13.7% in the healthy control group (*P* < 0.001). These data all suggest that in contrast to proinflammatory Th1 cytokines, MV induces a more robust and sustained Th2 cytokine activation locally in lungs, which might be predisposing patients receiving mechanical ventilation to having lung infections or increase the severity of infections when already infected.

### Bacterial challenge after mechanical ventilation leads to persistence of M2 macrophages and a worse disease outcome

MV prior to bacterial challenge (MV+Pa) significantly increased disease severity, bacterial load, and mortality compared to the Pa challenge alone (*P* < 0.05, Mantel-Cox log rank test) as reported previously, indicating that MV prior to infection contributes to pneumonia pathogenesis **(****Fig. 3A****).** Both pneumonia models were also characterized by significant increased neutrophilic infiltration that was non-significantly higher in the Pa group **(****Fig. 3B****).** To study whether MV-activated M2 macrophages drive pneumonia pathology, we studied and showed that rats receiving bacterial inoculum after MV had a significant 61% increase of arginase-1 (Arg1)-positive M2 macrophages 24 hours after infection compared to animals that received the same *P*. *aeruginosa* inoculum without MV (*P* < 0.05) **(****Fig. 6****).** Double immunocytochemistry revealed that MV+Pa animals carried 56% Arg1-CD68 double-positive cell population, very similar to the Arg1+ M2 macrophages proportion observed earlier for MV-24 group **(****Fig. 3C****),** compared to 36.5% observed for the Pa group (*P* < 0.001). Similarly, eosinophilic infiltration was 2.3 times increased in the MV+Pa group compared to the Pa group (P < 0.05) but remarkably similar to the MV-24 group **(****Fig. 1A** **and** **3B****).** These data suggest that mechanical ventilation activated M2 macrophages and Th2 cytokines drive VAP pathology despite the presence of Gram-negative bacteria - one of the strongest Th1 cytokine activators.

### Determination of ratio Th2/Th1 cytokines in lungs

Total RNA was extracted from lung tissue from animal ventilated for 2h and euthanized immediately after MV or after 24 h and fold differences were made compared to non-ventilated control animals. For each animal, the ratio IL4 (Th2 cytokine) and IFNγ (Th1 cytokine) was made (Fig. 7). Data is presented as group averages ± SEM for 6 animals per group. The ratio of IL4/IFNγ significantly increased after MV (**P* < 0.05 for both MVI and MV-24 groups) and could be used as a suited marker for detecting a Th2 shift that can lead to pneumonia development in mechanically ventilated patients.

### DISCUSSION

Mechanical ventilation is one of the biggest risk factors for VAP increasing the odds of developing pneumonia 10-fold. While the precise reason for this is unknown, both VAP and post-MV complications including organ failure are suggested to be due to increased proinflammatory cytokines such as IFNγ, TNFα, IL6, IL1α, and IL1β. Utilizing protective ventilation protocols utilized in clinics (tidal volume of 8 mL/kg), we indeed observe activation of all these proinflammatory cytokines. However, lack of stimulation of Th1-cell specific transcription factor *Tbet* (actually a significant down-regulation by ≈3 fold) and a drastic reduction in the levels of key proinflammatory cytokines such as IFNγ, TNFα and IL6 to normal levels in time periods as short as 24 hours post-MV leads credence to the fact that MV-driven proinflammatory cytokine surge is largely acute and does not have a long term consequence. This is supported by sporadic reports of largely unchanged or depressed levels of IFNγ, TNFα and IL6 production from stimulated blood cells from children ventilated for 2 h (Plotz, Vreugdenhil et al. 2002). By contrast, we show here that CD69, a pan T-cell activation marker was significantly upregulated in lungs suggesting that MV does drive activation of other T-cell lineages.

Firstly, the absence of increased *Tbet* expression and local proinflammatory cytokine secretion with moderate IFNγ expression levels allowed us to hypothesize that MV could trigger innate immune cells within the lung to secrete IL17 and IL22 as has been noticed in other conditions (Park, Li et al. 2005, Fujiwara, Hirose et al. 2007). Indeed, we show here the distinct involvement of IL17 and IL22 in VILI, whereby these cytokines were drastically elevated as a consequence of MV and remained elevated 24 hours after termination of MV. Both IL17 and IL22 have been implicated in airway inflammation in both pro- and anti-inflammatory pathways. For instance, IL17 secretion by γδ-T cells has shown to mediate resolution of allergic airway inflammation, however, is also shown to be detrimental in asthma by attracting neutrophils (Nembrini, Marsland et al. 2009). Similarly, a high expression of IL22 has been recently shown to suppress immune responses via an IL10 dependent pathway, however, it also aggravates pulmonary inflammation by IL33 mediated pathway. Functionally distinct subsets of pro- and anti-inflammatory Th17 cells are also described that are shown to interchange their phenotypes by transdifferentiation (Zhang, Goncalves et al. 2008). Not withstanding the precise mechanism and the precise Th17 subtype involved, the magnitude of the upregulation and the tenacious high levels of IL17a, IL17f and IL22 suggest that the IL17-family of cytokines are at least as important players as the classical proinflammatory cytokines in MV-related immune changes and VILI.

Secondly, we show here a Th2-driven immunosupressive response in lung tissue as a consequence of MV. IL4 and IL13 are the most important members of Th2 cytokine family. The levels of IL4 transcripts were upregulated ≈5 fold as a consequence of a protective MV protocol and remained significantly upregulated to ≈4 fold levels at 24 h post-MV. We confirmed these data on IL4 protein levels that were also more than 14-fold elevated in bronchoalveolar lavage fluid. IL13 transcript levels also began to elevate as a consequence of protective ventilation but became significant only after 24 hours when they were ≈4 fold elevated. Because IL13 is primarily associated with the induction of airway diseases (Venkayya, Lam et al. 2002) we ventilated rats with tidal volume of 30 mL/kg and showed that IL13 transcripts were elevated 80-fold immediately after the non-protective ventilation protocol. By comparison, proinflammatory TNFα was elevated to only 27 fold, suggesting that IL13 is also an important Th2 cytokine induced by mechanical ventilation.

As a consequence of protective ventilation, we also show here a robust and sustained significant elevation of IL18, an important inducer of IL4 and IL13 production by NK cells (Nakanishi, Yoshimoto et al. 2001) Moreover, both IL18 and IL4 potently induce naive CD4+ T cells differentiation into CD4+Th2 effector cells (Nakanishi, Yoshimoto et al. 2001). The increased expression of these Th2 anti-inflammatory cytokines also corroborates with the increased influx of eosinophils in the lung. Eosinophils are innate immune leukocytes elicited by Th2 cells and associated with the effector arm of Th2 immune responses. However, accumulating evidence over the past decade revealed a much more dynamic picture of Th2 immunity, where eosinophils are present very early in response to Th2-inducing agents and function in the initiation of Th2 immunity. These data suggest that MV activates Th2 cells leading to sustained production of Th2 cytokines IL4 and IL13 even after MV is terminated.

Interestingly, IL4 expression in lung is shown to have immunosuppressive actions whereby it increases the risk of pneumonia development in a mouse model (Kang, Rouse et al. 2009) and IL4 knock out mice have been shown to be more resistant towards secondary *P*. *aeruginosa* pneumonia development. Together with IL4, IL13 also induces macrophage polarization towards the Arg1+ immunosuppressive M2 phenotype (Knippenberg, Brumshagen et al. 2015). We show significant higher numbers of Arginase1+ M2 macrophages in ventilated animals, where despite strong co-presence of proinflammatory cytokines, 57% of all activated (CD68+) macrophages were Arg1+ compared to ≈14% in the healthy control group. Interestingly, IL13-Arg1+ immunosuppressive pathway in macrophages is shown to actively reduce lung protective immunity against infections such as *S*. *pneumoniae* (Knippenberg, Brumshagen et al. 2015).

Surprisingly, the significantly higher proportion of Arg1+ activated macrophages did not alter after bacterial instillation although bacteria are one of the strongest activators of M1 macrophages. This correlated well with the increased mortality and exaggerated lung pathology observed in VAP model of *P*. *aeruginosa* that also had a higher number of bacteria in lungs post mortem compared to a non-MV pneumonia model. Increased bacterial survival has been noticed earlier as a direct consequence of MV in animal models of. Although these mechanisms remain to be confirmed in VAP patients, our data suggest that MV-induced IL4/IL13 immune-depressive pathways render subjects more susceptible for bacterial infection and their appropriate targeting could be a viable therapeutic option for VAP. Notably, several phase II/III trials are currently evaluating the efficacies of antibodies targeting IL4 and/or IL13 in asthma patients (Danese, Rudzinski et al. 2015).

### REFERENCES

Conway Morris, A., Kefala, K., Wilkinson, T.S., Moncayo-Nieto, O.L., Dhaliwal, K., Farrell, L., Walsh, T.S., Mackenzie, S.J., Swann, D.G., Andrews, P.J., et al. (2010). Diagnostic importance of pulmonary interleukin-1 beta and interleukin-8 in ventilator-associated pneumonia. Thorax 65, 201-207.
Danese, S., Rudzinski, J., Brandt, W., Dupas, J.L., Peyrin-Biroulet, L., Bouhnik, Y., Kleczkowski, D., Uebel, P., Lukas, M., Knutsson, M., et al. (2015). Tralokinumab for moderate-to-severe UC: a randomised, double-blind, placebo-controlled, phase Ila study. Gut 64, 243-249.
Fujiwara, M., Hirose, K., Kagami, S., Takatori, H., Wakashin, H., Tamachi, T., Watanabe, N., Saito, Y., Iwamoto, I., and Nakajima, H. (2007). T-bet inhibits both TH2 cell-mediated eosinophil recruitment and TH17 cell-mediated neutrophil recruitment into the airways. The Journal of allergy and clinical immunology 119, 662-670.
Gagliani, N., Vesely, M.C., Iseppon, A., Brockmann, L., Xu, H., Palm, N.W., de Zoete, M.R., Licona-Limon, P., Paiva, R.S., Ching, T., et al. (2015). Th17 cells transdifferentiate into regulatory T cells during resolution of inflammation. Nature 523, 221-225.
Kang, C.I., Rouse, M.S., Patel, R., Kita, H., and Juhn, Y.J. (2009). Allergic airway inflammation and susceptibility to pneumococcal pneumonia in a murine model with real-time in vivo evaluation. Clinical and experimental immunology 156, 552-561.
Knippenberg, S., Brumshagen, C., Aschenbrenner, F., Welte, T., and Maus, U.A. (2015). Arginase 1 activity worsens lung-protective immunity against Streptococcus pneumoniae infection. European journal of immunology 45, 1716-1726.
Maria Cernada, J.E., Julia Kuligowski, Antonio Nunez, Elena Cubells, Marta Aguar, Maximo Vento (2015). IL-17 Concentration in Bronchoalveolar Lavage Fluid Obtained With a Blind-Protected Catheter Predicts Ventilator Associated Pneumonia in Newborn Infants. Abstract publishied in report of the Pediayric Academic Societies annual meeting.
Nakanishi, K., Yoshimoto, T., Tsutsui, H., and Okamura, H. (2001). Interleukin-18 is a unique cytokine that stimulates both Th1 and Th2 responses depending on its cytokine milieu. Cytokine & growth factor reviews 12, 53-72.
Nembrini, C., Marsland, B.J., and Kopf, M. (2009). IL-17-producing T cells in lung immunity and inflammation. The Journal of allergy and clinical immunology 123, 986-994; quiz 995-986.
Park, H., Li, Z., Yang, X.O., Chang, S.H., Nurieva, R., Wang, Y.H., Wang, Y., Hood, L., Zhu, Z., Tian, Q., et al. (2005). A distinct lineage of CD4 T cells regulates tissue inflammation by producing interleukin 17. Nature immunology 6, 1133-1141.
Plotz, F.B., Vreugdenhil, H.A., Slutsky, A.S., Zijlstra, J., Heijnen, C.J., and van Vught, H. (2002). Mechanical ventilation alters the immune response in children without lung pathology. Intensive care medicine 28, 486-492.
Schmittgen, T.D., and Livak, K.J. (2008). Analyzing real-time PCR data by the comparative C(T) method. Nature protocols 3, 1101-1108.
Venkayya, R., Lam, M., Willkom, M., Grunig, G., Corry, D.B., and Erie, D.J. (2002). The Th2 lymphocyte products IL-4 and IL-13 rapidly induce airway hyperresponsiveness through direct effects on resident airway cells. American journal of respiratory cell and molecular biology 26, 202-208.
Xia, Y.F., Liu, C.Q., Shi, H.J., and Ma, L. (2009). (Xia, Liu et al. 2009). Zhongguo dang dai er ke za zhi = Chinese journal of contemporary pediatrics 11, 645-648.
Xu, D., Trajkovic, V., Hunter, D., Leung, B.P., Schulz, K., Gracie, J.A., Mclnnes, I.B., and Liew, F.Y. (2000). IL-18 induces the differentiation of Th1 or Th2 cells depending upon cytokine milieu and genetic background. European journal of immunology 30, 3147-3156.

## Claims

1. A method for detecting a subject at risk of developing ventilator associated pneumonia (VAP), upon mechanical ventilation of said subject; said method comprising the steps of:
a) analysing the expression level of one or more Th2/Th17 cytokines selected from the list comprising IL17a/f, IL4, IL13 and IL22 in a test sample obtained from said subject;
b) comparing the expression level obtained in step a) with the expression level of said one or more Th2/Th17 cytokines in a reference sample;
wherein an increased level in said one or more Th2/Th17 cytokines in said test sample, compared to said control sample, is indicative of a subject having an increased risk of developing ventilator associated pneumonia upon mechanical ventilation of said subject.

2. The method according to claim 1; wherein said expression level is the absolute expression level of said Th2/Th17 cytokines.

3. The method according to claim 1; wherein said expression level is the relative expression level of said Th2/Th17 cytokines vs one or more Th1 reference cytokines selected from the list comprising IFNγ or IL6.

4. The method according to anyone of claims 1 to 3; wherein said test sample is obtained within about 1 h to about 48h after the start of mechanical ventilation of said subject; preferably within about 4h to about 24h after the start of mechanical ventilation of said subject.

5. The method according to anyone of claims 1 to 4; wherein said test sample is obtained before the first clinical manifestation of VAP, and after the start of mechanical ventilation of said subject.

6. The method according to anyone of claims 1 to 5; wherein said reference sample, is a sample obtained from the subject before the start of mechanical ventilation of said subject.

7. The method according to anyone of claims 1 to 6; wherein said samples are selected from the list comprising bronchoalveolar lavage fluid, endotracheal aspirates, whole blood, plasma and serum.

8. The method according to anyone of claims 1 to 7; wherein the expression level of said one or more interleukins is determined by protein assays or real-time quantitative PCR assays.

9. An interleukin-reducing compound for use in preventing or delaying onset of ventilator associated pneumonia in a subject undergoing mechanical ventilation; wherein said interleukin-reducing compound is selected from the list comprising:
- a compound capable of reducing expression levels of one or more interleukins selected from the list comprising IL4, IL13, IL17a/f and IL22;
- a compound capable of blocking receptors or co-receptors of one or more interleukins selected from the list comprising IL4, IL13, IL17a/f and IL22; or
- a combination thereof.

10. A method for preventing or delaying onset of ventilator associated pneumonia, upon mechanical ventilation of said subject; said method comprising administering to said subject an interleukin-reducing compound selected from the list comprising:
- a compound capable of reducing expression levels of one or more interleukins selected from the list comprising IL4, IL13, IL17a/f and IL22;
- a compound capable of blocking receptors or co-receptors of one or more interleukins selected from the list comprising IL4, IL13, IL17a/f and IL22; or
- a combination thereof.

11. The interleukin-reducing compound for use as defined in claim 9, or the method as defined in claim 10; wherein said interleukin-reducing compound is selected from the list comprising interleukin-reducing drugs, interleukin-neutralizing antibodies, and interleukin or interleukin receptor antagonists.

12. The interleukin-reducing compound for use as defined in claim 9, or the method as defined in claim 10; wherein said compound is administered by intravenous injection and/or via aerosol.

13. The interleukin-reducing compound for use as defined in claim 9, or the method as defined in claim 10; wherein said compound is administered within 4h to 48h after the start of mechanical ventilation of said subject, preferably within 4h to 24h after the start of mechanical ventilation of said subject.
